(19) Europäisches Patentamt
European Patent Office
Office européen des brevets

(11) EP 4 574 119 A1

(12) EUROPEAN PATENT APPLICATION

(43) Date of publication:
25.06.2025 Bulletin 2025/26

(21) Application number: 23219986.9

(22) Date of filing: 22.12.2023

(51) International Patent Classification (IPC):
A61F 13/49 (2006.01)    A61F 13/496 (2006.01)
A61L 15/20 (2006.01)

(52) Cooperative Patent Classification (CPC):
A61F 13/496; A61F 13/49003; A61F 13/49004;
D04B 1/243; D10B 2101/12; D10B 2401/13;
D10B 2403/0112; D10B 2501/02; D10B 2509/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Essity Hygiene and Health Aktiebolag
405 03 Göteborg (SE)

(72) Inventors:
• PALMQVIST, Lisa
405 03 GÖTEBORG (SE)
• ALENLJUNG, Susanne
405 03 GÖTEBORG (SE)

(74) Representative: Nederlandsch Octrooibureau
P.O. Box 29720
2502 LS The Hague (NL)

Remarks:
Claims 16 to 22 are deemed to be abandoned due to
non-payment of the claims fees (Rule 45(3) EPC).

(54) WASHABLE ABSORBENT UNDERGARMENT

(57)    A washable absorbent undergarment (1) comprises one or more fabric panels (2) and an integrated absorbent assembly (10) comprising a wearer facing top layer of a textile material (15), a garment facing moisture barrier layer (12), and one or more optional intermediate layers (11, 16) between the top layer (15) and the moisture barrier layer (12). The moisture barrier layer (12) comprises a woven or knitted fabric comprising activated carbon.

## Fig. 3

## Description

### Field of the invention

The present disclosure relates to washable absorbent undergarments comprising an integrated absorbent assembly, as well as to the absorbent assembly itself, that are intended for sanitary use in absorbing body fluids such as urine and vaginal fluids. The disclosure relates in particular to provisions in such garments for reducing odours.

### Background art

A washable absorbent undergarment may be worn for the purpose of absorbing body fluids such as urine and vaginal fluids. Such garments form an environmental and economical advantageous alternative to disposable sanitary napkins and disposable absorbent undergarments. A washable absorbent undergarment typically comprises fabric panels of woven or knitted materials. Conventionally such a washable absorbent undergarment will also comprise an integral absorbent assembly located in the crotch region of the wearer when the undergarment is worn. After use the undergarment is washed or laundered before reuse. Washable absorbent undergarments were initially mainly used for low to moderate volumes of body fluids but for sustainability reasons there is also demand for undergarments suitable for moderate to heavy flows of body fluids. An example of such a washable absorbent undergarment is found in WO2023169665A1.

Depending upon the intended use, the absorbent assembly may vary in size and construction. It may extend over a smaller or larger area of the garment, e.g. depending on whether it is intended for day-time or night-time use. It may also be thicker or thinner, with additional wicking, distribution, barrier and absorbing layers. Nevertheless, in addition to being functional from an absorbing perspective, the resulting undergarment must inspire confidence and be comfortable to wear for prolonged periods without the presence or spread of any undesirable odours.

### Summary of the invention

The present disclosure is based on the insight that there is a need for a washable absorbent undergarment and absorbent assembly able to alleviate the presence or spread of any undesirable odours occurring during use or after storage before washing.

The present disclosure provides a washable absorbent undergarment in accordance with claim 1 and an absorbent assembly according to claim 14. Further embodiments are set out in the dependent claims and in the following description. The disclosure thus concerns a washable absorbent undergarment comprising one or more fabric panels and an integrated absorbent assembly comprising a wearer facing top layer, a garment facing moisture barrier layer, and one or more optional intermediate layers between the top layer and the moisture barrier layer, wherein the moisture barrier layer comprises a woven or knitted fabric comprising activated carbon. The disclosure further concerns a washable absorbent assembly comprising a wearer facing top layer, a garment facing moisture barrier layer, and one or more optional intermediate layers between the top layer and the moisture barrier layer, wherein the moisture barrier layer comprises a woven or knitted fabric comprising activated carbon.

Activated carbon has been found to be particularly efficacious in absorbing undesirable odours and the location of such material within an article has been shown to be of importance.

Garments of various forms have previously been proposed that make use of the properties of activated carbon. These have included protective clothing such as disclosed in US7062788B2 for filtering toxic chemicals from the air to protect a wearer. Also protective underwear for counteracting flatulence has been proposed in US5593398. The underwear is generally impervious, except for a hole in the back area into which a porous filter comprising activated carbon is inserted.

As used herein, the term "absorbent undergarment" refers to garments that are worn and intended to be placed against the skin of the wearer with the intended function of absorbing and containing body fluids such as urine and vaginal fluids including menstrual fluid, without significant visible external trace. The undergarment may for example be underwear, underpants, a panty, or swimwear of fabric. The undergarment is intended for adult users but use for children is not excluded. The undergarment may be designed for male or female use. It will be understood that most conventional garments will have a limited degree of absorbency but that for conventional undergarments the containment of body fluids is not intended, and an unexpected leakage of any significant volume would be perceptible externally either as a damp patch or discoloration that may penetrate through an outer garment.

The absorbent undergarment and the absorbent assembly according to the present disclosure is washable, i.e. intended to be laundered or otherwise restored after use for repeated reuse as a sanitary article. By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g. to 30°C or 60°C. The term is also intended to mean that the absorbent undergarment can be washed and reused more than once. Washable may be understood as being suitable to undergo at least 10 washing cycles at a temperature of at least 30°C, without disintegrating. During the washing, the absorbent assembly releases the absorbed fluids, thereby regenerating the absorptive functioning, enabling the absorbent assembly to be reused for the same purpose again. A washable absorbent undergarment comprises woven or knitted fabrics in contrast to a disposable absorbent article not containing such fabrics. A disposable absorbent article is mainly of nonwovens, plastic films, cellulose fibers and superabsorbent particles and is not constructed to be washable and reusable and is thus for single use only.

The term "fabric" as used in the present disclosure may refer to single or multiple layers of a flexible planar substance constructed from solutions, fibers, yarns, or any combination. Fabrics include knitted or woven materials. Although other terms like material, fabric, and cloth are synonyms, textile is most used in the global textile complex. Fiber is any substance, natural or manufactured, with a high length-to-width ratio with suitable characteristics for processing into fabrics and is the smallest component, hairlike in nature, that can be removed from a fabric. Fibers make most fabrics. A yarn is a grouping of fibers that is twisted or laid together to form a continuous strand that can be made into a textile fabric.

The moisture barrier layer comprises or consists of a woven or knitted fabric comprising activated carbon. Fibers used in the weave or knit may be of filament, stretch-broken or staple type and may be present in any filamentary form, including fibres and yarns that may contain activated carbon filaments of any length. Such activated carbon weave or knit may be commercially produced by the activation of the filaments within an existing fabric, either chemically or thermally.

The woven or knitted fabric may be of any appropriate construction. Knitting is a method of constructing a fabric by interlocking a series of loops of one or more yarns. There are two major classes of knitting namely warp and weft or filling knitting, both of which are included. Weaving is a method or process of interlacing two yarns or sets of yarns of similar materials so that they cross each other at right angles to produce woven fabric. The warp yarns run lengthwise in the fabric and the filling threads (weft) or picks, run from side to side. Any appropriate weave may be applied, including plain weave, double weave, compound weave or the like. Jacquard is a system of weaving or knitting that utilizes a versatile pattern mechanism to produce intricate designs by using punch cards controlling the patterns produced. Jacquard knit may be valid for circular, flatbed, warp or weft knit.

In a washable absorbent garment it has been found to be important that the odor control not only capture dry, gaseous species but also odors arising from the fluid and newly generated gaseous contaminants trapped in or just leaving the fluid. A woven or knitted barrier layer comprising activated carbon has been shown to capture odors from both liquid and gaseous contaminants. Activated carbon in the microporous structure of a knitted or woven fabric will trap the liquid long enough to allow for capturing odors in a washable absorbent article.

The moisture barrier layer, comprising a woven or knitted fabric of activated carbon fibres, may be of any suitable thickness required by its function and may have a thickness of between 0.2 mm and 1.0 mm, such as between 0.2 and 0.6 mm, as measured with ISO 5084 (1996) "Determination of thickness of textiles and textile products". The actual value may depend at least partially on whether or not this weave or knit is the only component of the moisture barrier layer. An exemplary laminate of a weave or knit of activated carbon fabric together with a moisture impermeable material may be in the range 0.35 mm to 0.47 mm with the impermeable part of the laminate being about 0.02 mm.

The moisture barrier layer may consist of or consist essentially of the weave or knit of activated carbon. Activated carbon fibres, filaments and yarns are naturally hydrophobic. A closely woven or knit structure can thus already achieve significant water repellence and there may be no need to add anything further. The moisture barrier layer may comprise a moisture impermeable material laminated or coated onto the woven or knitted material comprising activated carbon. The moisture impermeable material may be provided at any appropriate location with respect to the fabric comprising activated carbon. It may be provided only at a garment facing side thereof. Suitable moisture impermeable materials may include thermoplastic films, such as polyurethane or the like.

The moisture barrier layer may have a surface weight of between 50 and 300 gsm, preferably between 75 gsm and 200 gsm. It will be understood that where the moisture barrier layer comprises an additional layer of moisture impermeable material, the above weights apply to the combined moisture barrier layer.

The moisture barrier layer may be provided with any suitable quantity of activated carbon. In general, this will be uniformly distributed over the absorbent assembly although it will be understood that it may also be possible to provide increased amounts of activated carbon at key locations. There may be no activated carbon elsewhere in the garment except in the moisture barrier layer.

The activated carbon may be derived from any suitable source, including natural materials or synthetic polymers. Natural raw materials may be preferred as precursors, being a greener and more sustainable alternative. In an embodiment, the activated carbon has a surface area of more than 800 $m^2$/g and less than 2000 $m^2$/g.

Although the moisture barrier layer may be impervious to liquid water, the moisture barrier layer may have a breathability or moisture vapor transmission rate (WVTR) of 800-2000 g. Breathability or moisture vapor transmission rate (WVTR) is measured by the rate at which water vapor passes through, in grams of water vapour per square meter of fabric per 24-hour period ($g/m^2$/d), often abbreviated to just "g", measured in standard method NWSP 070.5.R0 Mocon.

In preferred embodiments, the absorbent assembly is permanently attached to at least one of the one or more fabric panels. By "permanently attached", is meant that no portion is intended to be separated from the body fabric during, or after use. All portions of the absorbent assembly are thus intended to be laundered together with the garment. It is not excluded that protective and removable layers may be present prior to first time use.

The absorbent assembly may be attached to the fabric panels of the undergarment over its whole surface e.g. by lamination or adhesive. In other embodiments, the absorbent assembly may not be directly bonded to the fabric panels but instead may be attached to the fabric via auxiliary parts such as edging strips or reinforcement patches. In certain embodiments, the absorbent assembly is attached to the one or more fabric panels by one or more bonding members. These may include one or more of a front bond member, a rear bond member and side bond members.

The undergarment may be of any suitable form and reference above and in the following to 'garment' is not intended to mean other than an undergarment i.e. an innermost garment intended for being in contact with the body of a user. The garment has a waist opening and two leg openings. It is however not excluded that closure elements e.g. between the waist opening and the leg openings may be provided, allowing easy removal or donning of the garment. The undergarment may have various designs, such as briefs, boxer, hipster, high waist, string, Brazilian, or other suitable and conventional undergarment designs. The fabric panels may form a front region, a back region and an intermediate region extending between the front and back regions, the front region and the back region being joined at the sides such that the undergarment forms a waist opening and a pair of leg openings. A central longitudinal axis of the undergarment is defined along the one or more fabric panels of the undergarment from the back region and towards the front region and a transversal crotch axis of the undergarment is defined in a direction extending between the leg openings. The transversal crotch axis may be located at the narrowest point of the garment and/or may be located at the mid-point of the central longitudinal axis. The transversal crotch axis divides the intermediate region into a front intermediate region extending longitudinally between the transversal crotch axis and a front end of each leg opening, and a rear intermediate region extending longitudinally between the transversal crotch axis and a rear end of each leg opening. The transversal crotch axis is perpendicular to the central longitudinal axis and the top layer, optional intermediate layers and moisture barrier layer are superimposed along a height axis, that is perpendicular to the longitudinal axis and the transversal crotch axis.

The absorbent assembly may be located in the intermediate region. It may also be located only in the intermediate region i.e. it may not extend into the front region and back regions, which together form the waist region. The absorbent assembly may be located predominantly in the front intermediate region. Thus, more than 50% of the area of the assembly may be located in the front intermediate region, alternatively more than 60% or more than 70% or more than 80%, the remainder being located in the rear intermediate region, behind the transversal crotch axis. The absorbent assembly may be substantially symmetrical along the central longitudinal axis.

The shape and size of the reusable absorbent assembly may be such that at least a crotch section of said undergarment is covered during use. The crotch section is a surface section having a size and shape to cover some or all of the genital area of a user, i.e. a surface section shaped to fit in between the legs of a user, that has a length sufficient to extend at least from the mons pubis to the perineum and a width that varies along the length. The crotch section thus has a width substantially corresponding to a transversal width in between leg openings of an undergarment. Thus, the entire crotch section is protected from soiling by the reusable absorbent assembly.

The area of the garment in which the absorbent assembly is present may be adapted according to the particular garment and the intended use. The woven or knitted material comprising activated carbon may cover an area of at least 10 $cm^2$ of the garment, preferably between 10 $cm^2$ and 400 $cm^2$, or between 50 $cm^2$ and 100 $cm^2$.

The woven or knitted material comprising activated carbon may cover an area of at least 10 $cm^2$ of the absorbent assembly, preferably between 10 $cm^2$ and 400 $cm^2$, or between 50 $cm^2$ and 100 $cm^2$.

The absorbent assembly may be of any appropriate shape according to the intended use and the shape of the undergarment. It may be dog-bone shaped, having curved sides and slightly enlarged ends. It may be four-sided, having a front edge, a rear edge and two side edges. The side edges may be positioned to be coincident with respective leg openings. In this context, "coincident" is intended to mean substantially aligned with and adjacent to the side edges, within the bounds of normal manufacturing practices. The absorbent assembly or parts thereof may wrap around the side edges in a seam or vice-versa. The absorbent assembly may also end just short of the side edges and be covered by an edging strip.

The absorbent assembly may be rectangular. In this context, "rectangular" is intended to denote that the assembly has an outer contour that is a four-sided shape, having two pairs of sides that are generally parallel to one another. The rectangular assembly or its components can be easily cut multiple times from a main material source (often a large sheet or roll of fabric or foil material), with a single cutting line forming two edges of adjacent components. Thus, the use of rectangular shaped assemblies reduces the amount of cutting and minimizes waste. Furthermore, the cutting of rectangular contours from a main material source allows for multiple components to be cut directly adjacent to one another, without having to account for a positioning direction of each individual component in the manufacturing line. Thus, no energy is wasted during production to account for variation in direction of each component.

At least 75% of the total length of the edges of the at least one rectangular shaped absorbent assembly may be straight edges. Thus at least 75% of the outer contour of the rectangular shaped absorbent assembly conforms to the rectangular shape, thereby limiting the amount of material wasted, while allowing for some minimal shape variations in the layer that are required to assure wearer comfort and prevent the article from bulging during use.

The top layer and the moisture barrier layer may be substantially equal in size and shape and may both have an outer contour that forms an outer contour of the absorbent assembly. In this manner, protection against leakage is available over the same area that is also directly visible to the user, giving a good indication to the user of the leakage protection provided by the absorbent assembly. In other embodiments, the layers need not be co-extensive. The top layer may extend beyond the moisture barrier layer e.g. in order to connect to the fabric panels of the garment.

The absorbent assembly may optionally comprise one or more intermediate layers. The intermediate layers may be coextensive with the top layer or may be smaller in area such as to be covered by the top layer. The absorbent assembly may comprise an intermediate wicking layer.

An intermediate absorbent layer may be provided. The absorbent layer may comprise any suitable washable absorbent material capable of receiving and retaining a volume of the body fluids intended to be absorbed by the garment. The absorbent layer may comprise hydrophilic fibres and/or an open cell porous structure. In particular the absorbent assembly may comprise both an absorbent layer and a wicking layer. The wicking layer may be located between the absorbent layer and the top layer.

A number of absorbent layers may be provided e.g. stacked together. A first absorbent layer may have different properties e.g. material, thickness and/or area to a second absorbent layer. The second absorbent layer may have a transverse width that is larger than a transverse width of the first absorbent layer. The shape of the second absorbent layer may more closely match the shape of the top layer. The larger transverse width ensures that a minimum absorbency is present over a larger area than provided by the first absorbent layer. Furthermore, the difference in width prevents the stacking of layer-edges, that could otherwise result in unwanted bulging of the absorbent assembly. The second absorbent layer may be located between the first absorbent layer and the top layer. An advantage with this order is an absorbent assembly that more easily follows the shape of the body and thereby feels less bulky.

The intermediate layers may collectively be referred to as the core, being all layers located between the top sheet and the moisture barrier layer. A width of the core, seen in transversal direction, may be less than 95% of a corresponding width of the absorbent assembly. This ensures that the moisture barrier layer and top sheet can adequately cover the core, in particular the edges of the core. The top sheet and the moisture barrier layer may be adhered to one another around the core, effectively closing off the core and preventing leakage at the edges.

The absorbent assembly may be configured as a pad that is adapted for detachable connection to the panels of an undergarment. Thereto the absorbent assembly may comprise an adhesive layer or a hook patch on its garment facing surface or any other suitable manner of connection. Alternatively, the garment may be provided with a suitable pocket or recess for receiving the absorbent assembly.

## Brief description of the drawings

Embodiments of a washable absorbent undergarment according to the present disclosure will be described by way of example, with reference to the attached drawings, in which:

Fig. 1 shows a front view of an exemplary absorbent undergarment comprising an absorbent assembly;
Fig. 2 shows a top view of the undergarment of Fig. 1 in a flat laid-out state with the joints between the rear region and the front region removed;
Fig. 3 is a schematic cut-away view of a portion of the absorbent undergarment of Fig. 1; and
Fig. 4 is a cross-sectional view through the undergarment of Fig. 1 taken in the direction IV-IV in Fig. 3.

## Description of embodiments

Different aspects of the present disclosure will be described more fully hereinafter with reference to the enclosed drawings. The absorbent undergarment disclosed herein can, however be realized in many different forms, such as different sizes and absorption levels, and should not be construed as being limited to the aspects set forth herein. In all figures of the following detailed description, the same reference numerals will be used to indicate the same elements.

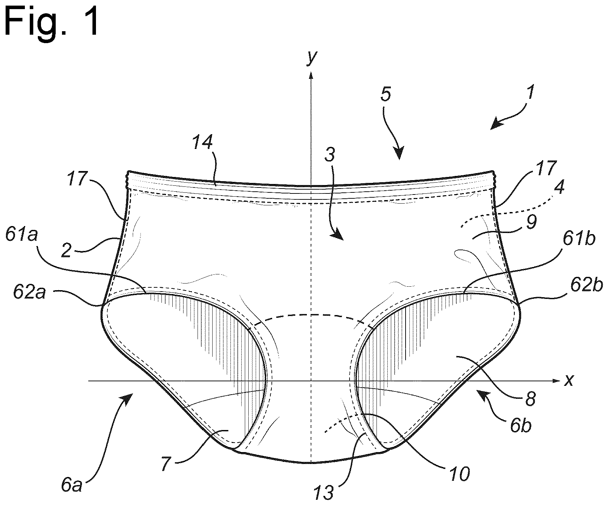

Fig. 1 illustrates as an example of a variant of a washable and reusable absorbent undergarment 1 in the form of a panty. As outlined in the above, the undergarment 1 as proposed herein may however have various designs, such as briefs, boxer, hipster, high waist, string, Brazilian, or other suitable undergarment designs. The undergarment 1 may be designed for male or female use.

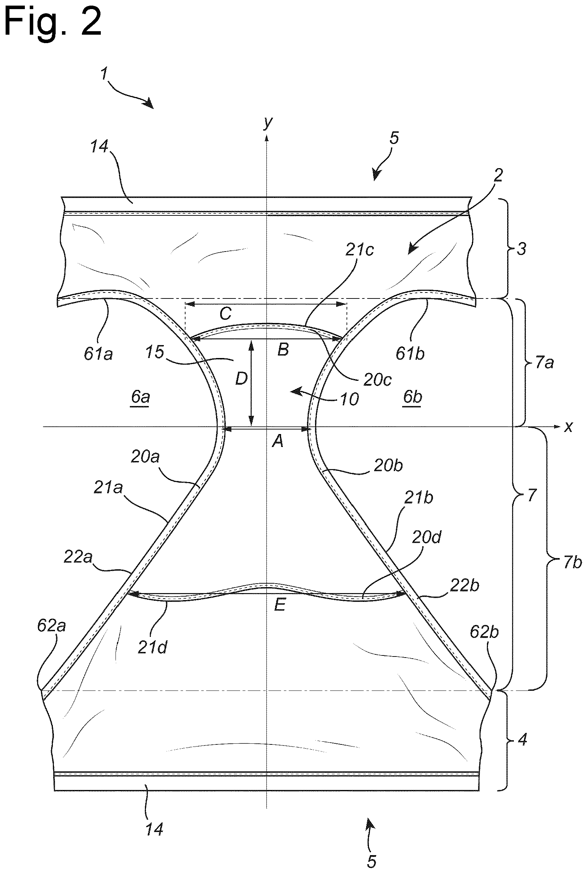

The undergarment 1 may comprise one or more fabric panels 2 forming a front region 3, a back region 4 and an intermediate region 7 extending between the front and back regions 3, 4. The front region 3 and the back region 4 are joined such that the undergarment 1 forms a waist opening 5 and a pair of leg openings 6a, 6b. As such, the front region 3 will be visible from the front of the user when the undergarment 1 is worn, and the back region 4 will be visible from the back of the user when the undergarment 1 is worn. The undergarment of the Fig. 1 and Fig. 2 example comprises one fabric panel 2, which is cut to form the front region 3, the back region 4 and the intermediate region 7. However, in other variants, a plurality of fabric panels may be joined so as to form the front region 3, the back region 4 and the intermediate region 7. For example, the undergarment 1 may comprise a front panel, a back panel and an intermediate panel. The one or more fabric panels may all comprise the same fabric, giving the undergarment a unitary appearance, or the one or more fabric may comprise different fabrics, for example to provide aesthetically pleasing undergarments comprising e.g. lace fabric.

The fabric of the one or more fabric panels 2 may be any suitable fabric, including naturally derived fibres and mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the fabric may be constructed of synthetic fibres or mixtures thereof selected from the group consisting of polyamide, acrylic, polyester. Further, the fabric may be constructed of a blend or a mixture of naturally derived and/or synthetic fibres. The fibres may be recycled fibres. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric may be breathable to allow vapor to escape from the wearer's skin and from the absorbent structure.

To join the front region 3 and the back region 4 so as to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or conventional mechanical bonds, such as stitching techniques.

A central longitudinal axis y of the undergarment 1 is defined along the one or more fabric panels 2 of the undergarment 1 from the back region 4 and towards the front region 3. As such, when an undergarment 1 is seen from the front side as in the example of Fig. 1, the central longitudinal axis y would be directed towards the waist opening 5 of the undergarment 1. However, when the undergarment 1 is seen from the back side (not shown), the central longitudinal axis y would be directed away from the waist opening 5 of the undergarment 1.

In Fig. 2, the absorbent undergarment 1 of Fig. 1 is shown in a flat laid-out state, where the side joints 17 of the undergarment 1 are removed. As seen in this laid-out state, the central longitudinal axis y is defined along the one or more fabric panels 2 and in a direction from the rear of the undergarment 1 towards the front of the undergarment 1. In Fig. 2, the absorbent undergarment 1 is shown from a wearer- facing side of the article 1. As illustrated in Fig. 1, the one or more fabric panels 2 will have an exterior side 9 facing away from the wearer, and an interior side 8 facing in a direction towards the wearer. Fig. 2 thus displays the undergarment 1 in a flat laid-out state as seen from the interior side 8.

Further, a transversal crotch axis x of the undergarment 1 is defined in a direction extending between the leg openings 6a, 6b and so as to divide the intermediate region 7 into a front intermediate region 7a extending longitudinally between the transversal crotch axis x and a front end 61a, 61b of each leg opening 6a, 6b, and a rear intermediate region 7b extending longitudinally between the transversal crotch axis x and a rear end 62a, 62b of each leg opening 6a, 6b, the transversal crotch axis x being perpendicular to the central longitudinal axis y.

As such, the transversal crotch axis x divides each leg opening 6a, 6b into a front portion of the leg opening 6a, 6b, which is intended to extend from the crotch of the user towards the front of the undergarment when the undergarment 1 is worn, and a rear portion of leg opening 6a, 6b, which is intended to extend from the crotch of the user towards the rear of the undergarment when the undergarment 1 is worn. As such, the transversal crotch axis x is to extend over a central crotch area of the user when the undergarment 1 is worn.

The absorbent undergarment 1 may comprise a waist band 14, arranged along the waist opening 5 of the absorbent undergarment 1. Although not shown, it will be understood that the waist band may be elasticated and may be provided with a frictional inner surface.

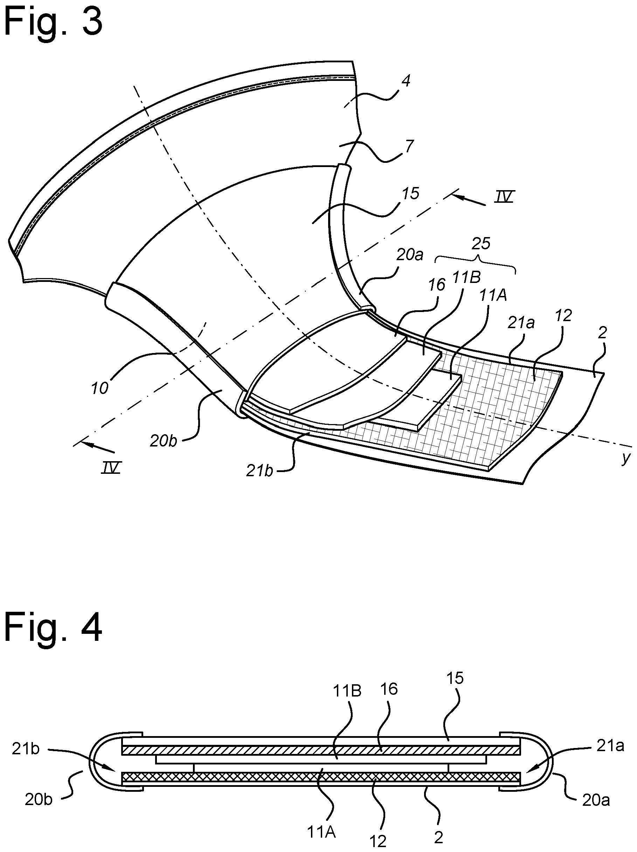

The undergarment 1 further comprises an absorbent assembly 10, to be described further below in the context of Fig. 3. The absorbent assembly 10 comprises a wearer facing top layer 15 that visibly illustrates to a user the extent of the absorbent assembly. As seen in Fig. 2, in the illustrated undergarment 1, the absorbent assembly 10 is located on an interior side 8 of the at least one fabric panel 2. The absorbent assembly 10 is arranged over the transversal crotch axis x and so as to extend in the front intermediate region 7a, and in the rear intermediate region 7b.

Although in some variants the absorbent assembly 10 may extend longitudinally beyond the front intermediate region 7a in a direction towards the front of the undergarment 1, in the illustrated variant, the absorbent assembly 10 extends no further than the front intermediate region 7a in the longitudinal direction towards the front region 3 of the undergarment 1. The longitudinal extension of the absorbent assembly 10 into the rear intermediate region 7b may depend for example on the design of the undergarment 1. In some variants, such as in the illustrated variant, the absorbent assembly 10 may extend no further than the rear intermediate region 7b in the longitudinal direction towards the rear region 4 of the undergarment 1.

As such, the absorbent assembly 10 may be confined to the intermediate region 7, wherein the need for absorbance is immediately prevalent. However, for example for undergarments intended primarily for night-time use, the absorbent assembly 10 may extend beyond the rear intermediate region 7b and into the rear region 4 of the undergarment 1, and/or beyond the front intermediate region 7a and into the front region 3 of the undergarment 1.

As exemplified by the illustrated undergarment 1, the absorbent assembly 10 forms a front edge 21c directed towards the front of the undergarment 1, a rear edge 21 d directed towards the rear of the undergarment, and a pair of side edges 21a, 21b. Each side edge 21a, 21b is at least partly directed towards a respective leg opening 6a, 6b. Each side edge 21a, 21b, thus connects the front edge 21c and the rear edge 21d.

Each side edge 21a, 21b may be at least partly arranged along the respective leg opening 6a, 6b of the undergarment 1, such as at the leg opening 6a, 6b of the undergarment 1. In some variants, and in the illustrated example, the entire side edges 21a, 21b are arranged at the respective leg openings 6a, 6b of the undergarment 1, i.e. the side edges 21a, 21b follow the contour of the leg openings 6a, 6b of the undergarment 1.

The shape of the absorbent assembly 10 may be adapted to various absorption needs and to various designs of the undergarment 1, so as to provide sufficient absorption and satisfactory fit.

For example, the side edges 21a, 21b may comprise concave portions, as seen towards the central longitudinal axis y. For example, as in the illustrated variant, the entire side edges 21a, 21b are concave towards the central longitudinal axis y, i.e. curving inwards towards the central longitudinal axis y. Thus, the absorbent assembly 10 may be adapted to fit between the wearer's legs. The front edge 21c of the absorbent assembly may similarly be shaped in view of the need for comfort and fit of the undergarment 1. In some variants, such as in the illustrated undergarment 1, the front edge may be convex from the transversal crotch axis x, i.e. curving away from the transversal crotch axis x. As illustrated in Fig. 2, at the location of the central longitudinal axis y, the front edge 21c may thus protrude towards the front of the undergarment 1 beyond the extension of the side edges 21a, b of the absorbent assembly 10. This may be beneficial for the fit of the undergarment to the body and contribute to the avoidance of unwanted creases in the fabric panels.

As mentioned in the above, each side edge 21a, 21b may at least partly be arranged at the respective leg opening 6a, 6b of the undergarment 1. This implies that the outer edge of the undergarment 1 forming each leg opening 6a, 6b at least partly comprises the side edge 21a, 21b of the absorbent assembly 10. For example, as in the illustrated variant, the outer edge of the undergarment 1 forming each leg opening 6a, 6b comprises the entire side edge 21a, 21b of the absorbent assembly 10. However, in other variants, the undergarment 1 may extend transversally beyond the absorbent assembly 10 in the directions towards each leg opening 6a, 6b in at least a part of the undergarment 1. For example, the side edges 21a, 21b of the absorbent assembly 10 may follow the leg openings 6a, 6b in a portion of the undergarment 1 adjacent the transversal crotch axis x, but the side edges 21a, 21b of the absorbent assembly 10 may deviate from the leg openings 6a, 6b in an area further to the front of the undergarment 1. In other words, the one or more fabric panels 2 may extend beyond the side edges 21a, 21b in directions away from the central longitudinal axis y. In such variants, the portions of the fabric panels 2 extending beyond the side edges 21a, 21b in directions away from the central longitudinal axis y may for example form leg portions of the undergarment 1. As such, the leg portions extending beyond the absorbent assembly 10 may adapt to and fold towards the wearer's legs, while the absorbent assembly 10 remains unfolded between the wearer's legs.

Further, when each side edge 21a, 21b is at least partly arranged at the respective leg opening 6a, 6b of the undergarment 1, as in the illustrated undergarment 1, a minimum assembly crotch width A of the absorbent assembly, i.e. a minimum distance between the side edges 21a, 21b of the absorbent assembly 10, may be equal to a minimum undergarment crotch width of the intermediate region 7 of the undergarment 1. In some undergarment designs suitable for the absorbent undergarment proposed herein, the intermediate region 7 displays a relatively narrower crotch portion, which crotch portion comprises the transversal crotch axis x, and relatively wider front and rear portions towards the front and rear, respectively, of the crotch portion. As such, the absorbent assembly 10 may have a minimum transversal width being the transversal assembly crotch width A at the transversal crotch axis x, and the width of the absorbent assembly 10 may increase along the longitudinal axis towards the front of the undergarment, from the minimum transversal assembly crotch width A to a front portion maximum width C. Also, the width of the absorbent assembly 10 may increase along the longitudinal axis y towards the rear of the undergarment 1, from the transversal assembly crotch width A to a rear portion maximum width.

The absorbent assembly 10 in Fig. 2 is permanently attached to at least one out of the one or more fabric panels 2 in at least part of the intermediate region 7 by a set of one or more bonding members 20a, 20b, 20c, 20d.

The bonding members 20a, 20b, 20c, 20d may have any configuration suitable for permanently attaching the absorbent assembly to the one or more fabric panels 2.

Further, the bonding members 20a, 20b, 20c, 20d may be any type of bonding member suitable for accomplishing the permanent attachment of the absorbent assembly 10 to the one or more fabric panels 2. As such, the set of bonding members 20a, 20b, 20c, 20d may comprise one or more adhesive bonding members, such as adhesive layers, strings or dots, and/or adhesive tape, or one or more mechanical bonding members, such as rivets or stitches, and/or a combination of adhesive and mechanical bonding members. However, adhesive bonding members, in particular elastic bonding film, and/or conventional mechanical bonding members such as conventional seams and stiches may be preferred.

The set of bonding members 20a, 20b, 20c, 20d could be applied for example as a bonding pattern or layer formed over the surface of the absorbent assembly 10 facing the one or more fabric panels 2. As such, the set of bonding members may comprise an adhesive layer or a pattern of adhesive dots or mechanical stiches spread over the area of the surface of the absorbent assembly 10 facing the one or more fabric panels 2. In another example, and as in the illustrated variant, the set of bonding members 20a, 20b, 20c, 20d may for example be arranged to permanently attach the absorbent assembly 10 to the at least one out of the one or more of the fabric panels 2 along at least part of one or more of the side edges 21a, 21b, the front edge 21c and the rear edge 21d.

For example, as in the illustrated variant, the set of bonding members 20a, 20b, 20c, 20d may be arranged to attach the side edges 21a, 21b, the front edge 21c and the rear edge 21d to the one or more fabric panels 2.

For example, and as in the illustrated undergarment 1, the set of bonding members may comprise one or more bonding lines 20a, 20b, 20c, 20d. As may be seen in the example of Fig. 2, one bonding line 20a, 20b may be arranged so as to extend along essentially each of the entire side edges 21a, 21b of the absorbent assembly 10. Further, in the illustrated example, one bonding line 20c is arranged to extend along essentially the entire front edge 21c and one bonding line 20d is arranged to extend along essentially the entire rear edge 20d.

Although in the illustrated variant, the bonding lines 20a, 20b, 20c, 20d are arranged to extend adjacent the respective edges 21a, 21b, 21c, 21d of the absorbent assembly 10, other variants may be envisaged where the bonding lines 20a, 20b, 20c, 20d are arranged at the respective edges 21a, 21b, 21c, 21 d, e.g. so as to extend over the respective edges 21a, 21b, 21c, 21d.

The one or more bonding lines 20a, 20b, 20c, 20d may, as in the illustrated example, be continuous bonding lines, such as seams or continuous adhesive lines. In other variants, however, the one or more bonding lines may be intermittent bonding lines.

The bonding members 20a, 20b, 20c, 20d may be arranged to directly attach the absorbent assembly 10 to the one or more fabric panels 2. Alternatively, the bonding members 20a, 20b, 20c, 20d may be arranged to indirectly attach the absorbent assembly 10 to the one or more fabric panels 2. For example, the bonding members 20a, 20b, 20c, 20d may be arranged to attach the absorbent assembly 10 to the one or more fabric panels 2 via an auxiliary element such as for example an edging strip or a reinforcement patch. Optionally, one or more of the bonding members 20a, 20b, 20c, 20d may be arranged so as to bond the layers of the absorbent assembly 10 together while attaching the absorbent assembly 10 to the one or more fabric panels 2.

Optionally, one or more of the bonding members 20a, 20b, 20c, 20d may be formed as part of e.g. a seam extending along an entire leg opening 6a, 6b of the intermediate portion.

In the illustrated variant, purely as an example, the intermediate region 7 is provided with a side edging 22a, 22b along each leg opening 6a, 6b, and the bonding members 20a, 20b provided along the side edges 21a, 21b of the absorbent assembly 10 are formed by parts of the seams connecting the side edgings 22a, 22b to the one or more fabric panels 2 of the intermediate region 7.

In the front intermediate region 7a of the undergarment 1, a maximum front bond width B over the set of bonding members 20a, 20b, 20c, 20d may be no more than 11 cm.

Thus, the maximum front bond width B is the maximum transversal width as measured over the set of bonding members 20a, 20b, 20c, 20d in the front intermediate region 7a. For example, and as in the illustrated variant, the maximum front bond width B may extend over the maximum transversal distance between a bonding line 20a along the first side edge 21a and a bonding line 20b along the second side edge 21b.

By restricting the maximum front bond width B, the one or more body panels 2 on the frontside of the undergarment 1 may adapt to the wearer's body without being unnecessarily hindered by the absorbent assembly 10, which, by virtue of the number of layers, is naturally less flexible than the one or more body panels 2. The maximum front bond width B is thus the maximum width along which the body panel or panels 2 in the front of the undergarment 1 will be restricted by the capacity of the absorbent assembly 10 to adapt to the wearer's body.

The maximum front bond width B may for example be no more than 10 cm, such as no more than 9 cm. Still, the maximum front bond width B may be selected to be sufficiently large so as to achieve sufficient absorption capacity. For example, the maximum front bond width B may be no less than 4 cm, such as no less than 5 cm.

For example, and as in the illustrated variant, the maximum front bond width B may be in the range from 4 to 11 cm, such as from 4 to 10 cm, or from 5 to 9 cm.

Further, and as illustrated by the example of Fig. 2, the absorbent assembly 10 may have a front portion maximum width C being the maximum transversal width of the absorbent assembly 10 in the front intermediate region 7a.

As intimated in the above, the set of bonding members 20a, 20b, 20c, 20d need not extend all the way to the side edges 21a, 21b of the absorbent assembly 10. Hence, the front portion maximum width C may be greater than the maximum front bond width B over the set of bonding members 20a, 20b, 20c, 20d. This implies that, at the location of the maximum front bond width B, the absorbent assembly 10 may extend beyond the set of bonding members 20a, 20b, 20c, 20d in the transversal directions away from the central longitudinal axis y. However, in this case the outmost transversal portions of the absorbent assembly 10 thus not being bonded to the one or more fabric panel 2 is not believed to severely restrict the capacity of the one or more fabric panels 2 to adapt to the wearer's body.

As such, the front portion maximum width C of the absorbent assembly 10 may be greater than the maximum front bond width B. For example, the front portion maximum width C may be no more than 12 cm, as compared to the maximum front bond width B being no more than 11 cm.

In some variants, as exemplified in the drawings, the difference between the maximum front bond width B and the front portion maximum width C may essentially result from a seam allowance provided peripheral of the set of bonding members 20a, 20b, 20c, 20d. For example, in some variants the front portion maximum width C and the maximum front bond width B may differ by less than 1 cm, such as less than 0.5 cm or less than 0.3 cm.

However, in other variants, the maximum front bond width B may be substantially equal to the front portion maximum width C of the absorbent assembly. For example, when the maximum front bond width B extends over a bonding line 20a at the first side edge 21a and a bonding line 20b at the second side edge 21b, the maximum front bond width B and the front portion maximum width C may coincide.

In some variants, the front portion maximum width C is no more than 11 cm, such as no more than 10 cm, such as for example no more than 9 cm. The front portion maximum width C in the front intermediary region 7a may be no less than 4 cm, such as no less than 5 cm. For example, the front portion maximum width C in the front intermediary region 7a may be in the range from 4 to 10 cm.

Further, the absorbent assembly 10 has a transversal assembly crotch width A being the transversal width of the absorbent assembly 1 at the longitudinal location of the transversal crotch axis x.

The transversal assembly crotch width A may be no greater than the maximum front bond width B and/or the front portion maximum width C.

For example, the absorbent assembly 10 may have a substantially constant transversal width along the front intermediate portion 7a.

However, to improve the fit of the undergarment 1 to the body, the transversal assembly crotch width A may be less than the front portion maximum width C of the absorbent assembly 10. Further, the transversal assembly crotch width A may be less than the maximum front bond with B of the absorbent assembly 10.

As intimated in the above, the shape of the intermediate region 7 of the undergarment 1 may in some variants deviate from the shape of the absorbent assembly 10 along the leg openings 6a, 6b, i.e. the one or more body fabric panels 2 may extend beyond the side edges 21a, 21b of the absorbent assembly 10 towards the leg openings 6a, 6b. As such, a transversal undergarment crotch width of the undergarment 1 at the longitudinal location of the transversal crotch axis x may differ from, e.g. be greater than, the transversal assembly crotch width A of the absorbent assembly 10.

In other variants, and as in the illustrated example, the transversal undergarment crotch width of the undergarment 1 at the longitudinal location of the transversal crotch axis x may be substantially equal to the transversal assembly crotch width A. In variants where the intermediate portion 7 comprises a relatively narrower crotch portion and relatively wider front and rear portions, as for example in the illustrated variant, the transversal undergarment crotch width of the undergarment 1 may be a minimum transversal width of the intermediate region 7 of the undergarment 1.

For example, the transversal assembly crotch width A may be the range from 3 to 8 cm.

Further, a first front portion length D of the absorbent assembly 10 may be defined along the longitudinal direction y from the transversal crotch axis x to the longitudinal position of the maximum front bond width B over the set of bonding members 20a, 20b, 20c, 20d in the front intermediate region 7a. As such, the first front portion length D indicates the distance between the transversal crotch axis x and the maximum front bond width B.

The first front portion length D may be less than 10 cm, such that in the range from 4 to 10 cm. As such, the shape and location of the absorbent assembly 10 in the undergarment 1 may be adapted so as to be useful for absorbing a moderate to heavy flow of body fluid without requiring an unnecessary large absorbent assembly 10. In the rear intermediate region 7b, a maximum rear bond width E being the maximum transversal width as measured over the set of bond members 20a, 20b, 20c, 20d in the rear intermediate region 7b may be defined. Due to anatomy, the formation of unwanted creases in the undergarment 1 is however not as prevalent on the rear side of the undergarment 1 as on the front side of the undergarment 1. Accordingly, the rear portion maximum width E may be more freely chosen so as to provide the desired absorption capacity and still provide satisfactory body fit towards the rear side of the wearer's body. Similarly, a maximum transversal rear width being the maximum transversal width of the absorbent assembly 10 in the rear intermediate region 7b may be relatively freely chosen.

As such, the maximum rear bond width E may be no less than the maximum front bond width B, and may for many variants be greater than the maximum front bond width B.

For example, and as in the illustrated variant, the absorbent assembly 10 may extend longitudinally towards the rear of the undergarment 1 no further than the rear intermediate region 7b. That is, the absorbent assembly 10 does not extend into the rear region 4 of the undergarment 1. As such, in these variants, the absorbent assembly 10 may be confined to the parts of the undergarment where it will be most useful for absorbing bodily fluids.

The total longitudinal length of the absorbent assembly 10 may be configured depending on e.g. the model of the undergarment and taking the need for sufficient absorption into account.

In some variants, when the set of bonding members comprises a mechanical bonding member, the mechanical bonding member may be arranged so as to extend through the absorbent assembly 10 and the at least one fabric panel 2. As such, the mechanical bonding member may be visible from the exterior side of the fabric panel 2.

Fig. 3 shows a schematic cut-away of part of the absorbent undergarment 1 of Figs 1 and 2 showing the back region 4 and part of the intermediate region 7. Fig. 4 shows a view in the direction IV-IV of Fig. 3. The absorbent assembly 10 is cut-away to show that beneath the top layer 15 is a first core layer 11A, a second core layer 11B, a wicking layer 16 and a moisture barrier layer 12. The first core layer 11A, second core layer 11B and wicking layer 16 may be termed intermediate layers and together form a core 25. Although in the variant of Fig. 3, three intermediate layers are shown, it will be understood that different numbers of layers may be provided as discussed elsewhere.

The top layer 15 comprises a water-permeable material thus allowing the body fluids to migrate to the underlying core 25. The top layer 15 may be constructed of any suitable fabric, including naturally derived fibres selected from the group consisting of cotton, wool, silk, cellulose, regenerated cellulose, rayon, viscose, modal, lyocell, Tencel, bamboo, hemp, flax, ramie, coir or banana. Alternatively, the top layer may be constructed of synthetic fibres selected from the group consisting of polyamide, acrylic, polyester, or elastane, such as a mixture of polyester and elastane. Further, the top layer 15 may be constructed of a blend or a mixture of naturally derived and/or synthetic fibres. The materials used for construction of the top layer should be soft and non-irritating to the skin and be readily penetrated by any body fluids. The top layer is washable and of a textile material, such as a knitted material. The top layer may comprise between 20% and 100% naturally derived fibres, more preferably between 40% and 100% naturally derived fibres and most preferably between 60% and 100% naturally derived fibres. The top layer 15 may have a basis weight of 80-200 gsm.

The wicking layer 16 may be provided directly underneath the top layer 15. The wicking layer 16 has wicking features to allow the moisture to spread away from the wearer and into the second core layer 11B. Wicking enables an efficient spread of the moisture, allowing the moisture to be received into a wider area of the underlying one or more core layers 11A, 11B. This feature is particularly relevant for users suffering from stress incontinence as spurts of urine may cause the core layers 11A, 11B to be saturated rapidly at the point of impact. By spreading the volume of the fluid over a wider receiving area in the core 25, the wicking features of the wicking layer 16 increase the overall absorptive capacity of the core 25. The wicking layer 16 may be constructed of any suitable fabric, including naturally derived fibres or mixtures thereof selected from the group consisting of cotton, wool, silk, cellulose, regenerated cellulose, rayon, viscose, modal, lyocell, Tencel, bamboo, hemp, flax, ramie, coir or banana. Alternatively, the wicking layer 16 may be constructed of synthetic fibres or mixtures thereof selected from the group consisting of polyamide, acrylic, polyester or elastane. Further, the wicking layer 16 may be constructed of a blend or a mixture of naturally derived and/or synthetic fibres. According to the present disclosure, the wicking layer should be launderable. The wicking layer 16 may comprise between 20% and 100% naturally derived fibres, more preferably between 40% and 100% naturally derived fibres and most preferably between 60% and 100% naturally derived fibres. The basis weight of the wicking layer may be 180-250 gsm.

The absorbent assembly 10 of the illustrated absorbent undergarment 1 contains two absorbent core layers 11A, 11B, each capable of absorbing liquid and releasing the liquid during laundering. Release of the absorbed liquid is beneficial as it enables the absorbent undergarment 1 to be restored for reuse. Reusable absorbent undergarments provide a more sustainable alternative to the commonly used disposable hygiene articles that are not intended to be reused.

In the illustrated embodiment, the second core layer 11B is substantially coextensive with the wicking layer 16. The wicking layer is slightly smaller in area than the top layer 15. The first core layer 11A is narrower than the second core layer 11B, ensuring additional absorptive capacity along the central longitudinal axis y of the garment 1.

The absorbent core layers 11 comprise any material capable of absorbing fluid, such as woven or nonwoven microfibre or polymer knits, fabric formed from hydrophilic fibres, absorbent fibres or powders. The absorbent core layers 11 may be of natural or synthetic fibres as described above for the top layer 15 and wicking layer 16 but may alternatively be of polyester and polyamide.

The absorbent core layers 11A, 11B may also comprise a material having an open cell porous structures such as high loft or synthetic fibres having reservoir properties. The core layers may comprise between 20% and 100% naturally derived fibres, more preferably between 40% and 100% naturally derived fibres and most preferably between 60% and 100% naturally derived fibres. The basis weight of each core layer 11A, 11B may be 200-350 gsm.

The moisture barrier layer 12 comprises a woven or knitted material comprising activated carbon of any suitable construction to prevent liquid from migrating from the absorbent assembly 10 to the fabric panel 2. The moisture barrier layer 12 in the illustrated variant, is a tightly woven activated carbon cloth and sufficiently impermeable by nature. One suitable material is Flexzorb ® from Chemviron SA. Laminates with polymer films may also be used. The moisture barrier layer 12 may comprise a coating of a moisture-impermeable material on the rear garment facing surface of the woven or knitted material comprising activated carbon. The coating may be a polymer such as urethane wax or polyurethane provided for example on the surface facing away from the wearer of the moisture barrier layer 12.

The moisture barrier layer 12 is also preferably breathable, to allow vapour to escape from the absorbent undergarment 1 while preventing liquid from passing through the fabric layer. Air and vapour permeability may be achieved by the woven or knitted material comprising activated carbon or by an additional rate controlling layer attached thereto.

Also shown in Fig. 3 and Fig 4, are bonding members 20a and 20b, whereby the absorbent assembly 10 is joined to the fabric panel 2 using elastic bonding film that wraps around the first and second side edges 21a, 21b. It will be understood that other options for forming the bonding members may also be used.

**Example - Measurement of reduction in odorous substances**

Samples were prepared by cutting circular pieces (ø 4.5 cm) of different materials and stacking the materials. The prepared samples are described in Table 1.

Table 1. Samples

| Sample no. | Content |
|---|---|
| 0 | Reference - A stack of two absorbent polyester terry fabric layers (each layer of 320 gsm) |
| 1 | A stack of two absorbent polyester terry fabric layers (each layer of 320 gsm) + one layer of Chemviron FLEXSORB® FM30K 100% activated carbon fabric (110 gsm) positioned below the absorbent fabric layers |
| 2 | A stack of two absorbent polyester terry fabric layers (each layer of 320 gsm) + one layer of Chemviron FLEXSORB® FM30K 100% activated carbon fabric (110 gsm) positioned below the absorbent fabric layers - identical to Sample 1 and further washed 10 times before the test |

The samples were placed in 500-ml Duran bottles, after which 2 ml of a test liquid was added to each sample. The test liquid contained four odorous substances: 2,3-butanedione, 3-methylbutanal, dimethyl disulphide, dimethyl trisulphide. The Duran bottles were sealed. After equilibration for 4 hours in 35°C, the odorous substances in the headspace above the sample were sampled and analyzed by ATD-GC-MS/FID. The headspace concentrations of odorous substances were determined.

The reduction of headspace concentration of odorous substance is calculated for each odorous substance from the equation:

$$\text{Reduction in odorous substances} = (1 - C_{test}/C_{ref}) \times 100$$

where:

$C_{test}$ = Headspace concentration of the test sample
$C_{ref}$ = Headspace concentration of the reference sample

The calculated reductions in headspace concentration of sample 1 and 2 respectively, compared to sample 0 are shown in table 2.

Table 2. Calculated reduction of odorous substances compared to sample 0

| Odorous substance | Sample 1 | Sample 2 |
|---|---|---|
| 2,3-Butanedione | 100% | 100% |
| 3-Methylbutanal | 90% | 80% |
| Dimethyl disulphide | 100% | 98% |
| Dimethyl trisulphide | 100% | 100% |

The results show that substantial reductions in odour can be achieved and maintained after washing using activated carbon for a number of odorous substances.

The disclosure may be varied within the scope of the appended claims. For example, the materials and dimensions used for the different layers forming the absorbent insert may be varied, as indicated above.

**Claims**

1.   A washable absorbent undergarment (1), comprising one or more fabric panels (2) and an integrated absorbent assembly (10) comprising a wearer facing top layer (15), a garment facing moisture barrier layer (12), and one or more optional intermediate layers (11, 16) between the top layer (15) and the moisture barrier layer (12), wherein the moisture barrier layer (12) comprises a woven or knitted fabric comprising activated carbon.

2. The garment of claim 1, wherein the moisture barrier layer (12) comprises or consists of a weave or knit of activated carbon fibres.

3. The garment of claim 1 or 2, wherein the moisture barrier layer (12) has a thickness of between 0.2 mm and 1.0 mm, as measured with ISO 5084 (1996).

4. The garment according to any one of the preceding claims, wherein the moisture barrier layer (12) comprises a moisture impermeable material laminated or coated onto the woven or knitted material comprising activated carbon.

5. The garment of claim 4, wherein the moisture impermeable material is a thermoplastic film, such as polyurethane.

6. The garment according to any one of the preceding claims, wherein the moisture barrier layer (12) has a basis weight of between 50 gsm and 300 gsm.

7. The garment according to any one of the preceding claims, wherein the activated carbon has a surface area of more than 800 $m^2/g$ and less than 2000 $m^2/g$.

8. The garment according to any one of the preceding claims, wherein the woven or knitted material comprising activated carbon covers an area of at least 10 $cm^2$ of the garment, such as between 10 $cm^2$ and 400 $cm^2$, or between 50 $cm^2$ and 100 $cm^2$.

9. The garment according to any one of the preceding claims, wherein the moisture barrier layer (12) has a moisture vapor transmission rate (WVTR) of between 800g and 2000 g, as measured with NWSP 070.5.RO Mocon.

10. The garment according to any one of the preceding claims, wherein the absorbent assembly (10) is permanently attached to at least one of the one or more fabric panels (2).

11. The garment according to claim 10, wherein the absorbent assembly (10) is attached to the one or more fabric panels (2) by one or more bonding members (20), optionally comprising one or more of a front bond member, a rear bond member and side bond members.

12. The garment according to any one of the preceding claims, wherein the fabric panels (2) form a front region (3), a back region (4) and an intermediate region (7) extending between the front and back regions (3, 4), the front region (3) and the back region (4) being joined such that the undergarment (1) forms a waist opening (5) and a pair of leg openings (6a, 6b), wherein a central longitudinal axis (y) of the undergarment (1) is defined along the one or more fabric panels (2) of the undergarment (1) from the back region (4) and towards the front region (3), and a transversal crotch axis (x) of the undergarment (1) is defined in a direction extending between the leg openings (6a, 6b) and so as to divide the intermediate region (7) into a front intermediate region (7a) extending longitudinally between the transversal crotch axis (x) and a front end (61a, 61b) of each leg opening (6a, 6b), and a rear intermediate region (7b) extending longitudinally between the transversal crotch axis (x) and a rear end (62a, 62b) of each leg opening (6a, 6b), the transversal crotch axis (x) being perpendicular to the central longitudinal axis (y), the top layer, optional intermediate layers and moisture barrier layer being superimposed along a height axis (z), perpendicular to the longitudinal axis (y) and the transversal crotch axis (x).

13. The garment according to claim 12, wherein the absorbent assembly (10) is located in the intermediate region (7).

14. A washable absorbent assembly (10) comprising a wearer facing top layer (15), a garment facing moisture barrier layer (12), and one or more optional intermediate layers (11, 16) between the top layer (15) and the moisture barrier layer (12), wherein the moisture barrier layer (12) comprises a woven or knitted material comprising activated carbon.

15. The assembly of claim 14, wherein the moisture barrier layer (12) comprises or consists of a weave or knit of activated carbon fibres.

16. The assembly according to claim 14 or claim 15, wherein the moisture barrier layer (12) comprises a moisture impermeable material laminated or coated onto the woven or knitted material comprising activated carbon.

17. The assembly according to claim 16, wherein the moisture impermeable material is a thermoplastic film, such as polyurethane.

18. The assembly according to any one of claims 14 to 17, wherein the moisture barrier layer (12) has a basis weight of between 50 gsm and 300 gsm.

19. The assembly according to any one of claims 14 to 18, wherein the activated carbon has a surface area of more than 800 $m^2$/g and less than 2000 $m^2$/g.

20. The assembly according to any one of claims 14 to 19, wherein the woven or knitted material comprising activated carbon covers an area of at least 10 $cm^2$, such as between 10 $cm^2$ and 400 $cm^2$, or between 50 $cm^2$ and 100 $cm^2$ of the assembly.

21. The assembly according to any one of claims 14 to 20, wherein the moisture barrier layer (12) has a moisture vapor transmission rate (WVTR) of between 800g and 2000 g, as measured with NWSP 070.5.RO Mocon.

22. The assembly according to any one of claims 14 to 21, wherein the absorbent assembly (10) comprises releasable fastening elements on a garment facing surface.

# Fig. 1

# Fig. 2

# Fig. 3

# Fig. 4

Europäisches
Patentamt

European
Patent Office

Office européen
des brevets

# EUROPEAN SEARCH REPORT

Application Number

EP 23 21 9986

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2013/045651 A1 (ESTEVES DE SOUSA FANGUEIRO RAUL MANUEL [PT] ET AL) 21 February 2013 (2013-02-21) * paragraphs [0001], [0011], [0012], [0016], [0042]; claims 1-7 * | 1-15 | INV. A61F13/49 A61F13/496 A61L15/20 |
| Y | US 2003/163106 A1 (BLANEY CAROL ANN [US] ET AL) 28 August 2003 (2003-08-28) * paragraphs [0043], [0061] - [0063]; figures 1, 2 * | 1-15 | |
| A | WO 2015/156686 A2 (RSD HOLDINGS LTD [NZ]) 15 October 2015 (2015-10-15) * claims 1-7; figure 1 * | 1-15 | |
| Y | CN 219 070 986 U (SICHUAN AIYISHENG MEDICAL TECH CO LTD) 26 May 2023 (2023-05-26) * claims 1-10; figures 1, 2; examples 1-5 * | 3,7 | |
| A | US 2021/290447 A1 (SEPELLO CASSANDRA A [US] ET AL) 23 September 2021 (2021-09-23) * paragraph [0005]; figures 1, 2A, 2B; examples * | 1-15 | TECHNICAL FIELDS SEARCHED (IPC)  A61F A61L |

The present search report has been drawn up for all claims

| Place of search | Date of completion of the search | Examiner |
|---|---|---|
| Munich | 29 July 2024 | Adechy, Miriam |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                                

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 23 21 9986

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2024

| Patent document cited in search report | Publication date | Patent family member(s) | Publication date |
|---|---|---|---|
| US 2013045651 A1 | 21-02-2013 | BR 112012022285 A2 | 15-05-2018 |
| | | CN 102933183 A | 13-02-2013 |
| | | DK 2542197 T3 | 19-07-2021 |
| | | EP 2542197 A1 | 09-01-2013 |
| | | ES 2881660 T3 | 30-11-2021 |
| | | HU E054757 T2 | 28-09-2021 |
| | | PL 2542197 T3 | 06-12-2021 |
| | | PT 2542197 T | 09-08-2021 |
| | | RU 2012137706 A | 10-04-2014 |
| | | US 2013045651 A1 | 21-02-2013 |
| | | WO 2011108954 A1 | 09-09-2011 |
| US 2003163106 A1 | 28-08-2003 | AR 025871 A1 | 18-12-2002 |
| | | AU 7092000 A | 30-04-2001 |
| | | BR 0014307 A | 21-05-2002 |
| | | CN 1376045 A | 23-10-2002 |
| | | EP 1231880 A1 | 21-08-2002 |
| | | JP 2003510131 A | 18-03-2003 |
| | | KR 20020035618 A | 11-05-2002 |
| | | MX PA02002216 A | 30-09-2002 |
| | | US 2003163106 A1 | 28-08-2003 |
| | | WO 0122908 A1 | 05-04-2001 |
| | | ZA 200201604 B | 30-04-2003 |
| WO 2015156686 A2 | 15-10-2015 | AU 2015202737 A1 | 05-11-2015 |
| | | AU 2017254823 A1 | 23-11-2017 |
| | | AU 2019204873 A1 | 25-07-2019 |
| | | CA 2945296 A1 | 15-10-2015 |
| | | CN 106456385 A | 22-02-2017 |
| | | EP 3128970 A2 | 15-02-2017 |
| | | JP 7009210 B2 | 25-01-2022 |
| | | JP 2017513671 A | 01-06-2017 |
| | | WO 2015156686 A2 | 15-10-2015 |
| CN 219070986 U | 26-05-2023 | NONE | |
| US 2021290447 A1 | 23-09-2021 | CN 115038417 A | 09-09-2022 |
| | | EP 4096607 A1 | 07-12-2022 |
| | | US 2021290447 A1 | 23-09-2021 |
| | | WO 2021155397 A1 | 05-08-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023169665 A1
- US 7062788 B2
- US 5593398 A